# EUROPEAN PATENT APPLICATION

(11) **EP 3 501 306 A1**
(43) Date of publication of application: **26.06.2019**
(21) Application number: 17845365.0
(22) Date of filing: 28.08.2017
(51) Int. Cl.: A24F 47/00

(54) **ATOMIZING ASSEMBLY, ATOMIZER AND ELECTRONIC CIGARETTE**

(30) Priority: 30.08.2016 CN 201620996260 U; 30.08.2016 CN 201610771329; 13.09.2016 CN 201621053925 U
(71) Applicant: Changzhou Jwei Intelligent Technology Co., Ltd., Changzhou, Jiangsu 213125 (CN); Joyetech Europe Holding GmbH, 6303 Zug (CH)
(72) Inventor: QIU, Weihua, Jiangsu 213125 (CN)
(74) Representative: Zaboliene, Reda
(86) International application number: PCT/CN2017/099268
(87) International publication number: WO 2018/041063

(57) **Abstract**

The present disclosure provides an atomizer, including an atomizing assembly and an intake ring sleeved at a lower portion of the atomizing assembly, the atomizing assembly includes a mounting seat, a liquid absorbing member and a heating member, the mounting seat includes a receiving portion, the receiving portion is recessed with a mounting portion, the intake ring is detachably sleeved on the receiving portion, an atomizing chamber is formed between the groove wall of the mounting portion and the inner wall of the intake ring, the liquid absorbing member and the heating member are detachably mounted on the mounting portion. The atomizer of the present disclosure realizes the purpose of quickly changing the liquid absorbing member and the heating member, has a simple structure and is convenient to operate. The present disclosure further provides an electronic cigarette having the above-mentioned atomizer.

## Description

### FIELD

The present disclosure relates to the field of electronic cigarette technology, and more particularly to an atomizing assembly, an atomizer and an electronic cigarette.

### BACKGROUND

The electronic cigarette includes an atomizer, the core component of the atomizer is an atomizing assembly. The atomizing assembly is provided with a liquid absorbing member for introducing the cigarette liquid and a heating member for heating the cigarette liquid under the driving of the power source device. Under the action of high temperature, the heating member will age, resulting in lower heating capacity and easy breakage. The liquid absorbing member is generally cotton, which is prone to coking at high temperature. Therefore, the heating member and the liquid absorbing member are both consumables. If the atomizing assembly is replaced as a whole, it will result in waste of reusable components in the atomizing assembly. The conventional heating member is a machine-wound type, the atomizing assembly is a disposable product, thus it is unable to replace or difficult to replace the liquid absorbing member and the heating member therein.

### SUMMARY

Based on this, it is necessary to provide an atomizer capable of quickly replacing the heating member and the liquid absorbing member, and an electronic cigarette thereof.

One technical solution of the present disclosure which is adopted to solve the technical problems is that: an atomizer includes an atomizing assembly and an intake ring sleeved at a lower portion of the atomizing assembly, the atomizing assembly includes a mounting seat, a liquid absorbing member and a heating member, the mounting seat includes a receiving portion, the receiving portion is recessed with a mounting portion, the intake ring is detachably sleeved on the receiving portion, an atomizing chamber is formed between the groove wall of the mounting portion and the inner wall of the intake ring, the liquid absorbing member and the heating member are detachably mounted on the mounting portion.

Furthermore, a side wall of the receiving portion is recessed inwardly to form the mounting portion. One end surface of the liquid absorbing member is closely attached to the bottom wall of the mounting portion, the heating member is in close contact with the end surface of the liquid absorbing member away from the bottom wall of the mounting portion. The atomizing assembly further includes a conducting mechanism. The conducting mechanism includes a second electrode mounted on the mounting portion. One end of the heating member is detachably connected to the mounting portion, the opposite end of the heating member is detachably connected to the second electrode.

Furthermore, the atomizer further includes a liquid storage assembly sleeved on an upper portion of the atomizing assembly. The liquid storage assembly is provided with a liquid storage chamber therein, the receiving portion is provided with a first liquid inlet groove in communication with the liquid storage chamber and the liquid absorbing member.

Furthermore, the mounting seat further includes an air outlet portion protruding from the upper end surface of the receiving portion. The liquid storage assembly includes a reservoir tube sleeved outside the air outlet portion, the liquid storage chamber is formed by a space between the inner wall of the reservoir tube and the outer wall of the air outlet portion.

Furthermore, the first liquid inlet groove is defined along the axial direction of the receiving portion, one end of the first liquid inlet groove passes through the upper end surface of the receiving portion and is in communication with the liquid storage chamber, a liquid inlet hole is defined at the bottom wall of the mounting portion, the other end of the first liquid inlet groove is in communication with the liquid absorbing member through the liquid inlet hole. Or, the first liquid inlet groove includes a first section and a second section, the first section of the first liquid inlet groove is defined along the axial direction of the receiving portion, the second section of the first liquid inlet groove is defined in the radial direction of the receiving portion, one end of the first section of the first liquid inlet groove is in communication with one end of the second section of the first liquid inlet groove, the opposite end of the first section of the first liquid inlet groove extends through the upper end surface of the receiving portion and is in communication with the liquid storage chamber, the other end of the second section of the first liquid inlet groove extends through the bottom wall of the mounting portion and is in communication with the liquid absorbing member.

Furthermore, an air outlet passage is defined in the air outlet portion along the axial direction of the air outlet portion, a second intake hole is defined at the side wall of the intake ring, both the second intake hole and the air outlet passage are in communication with the atomizing chamber.

Furthermore, the lower end surface of the receiving portion is provided with a first intake hole, the second intake hole is defined at the outer peripheral surface of the bottom wall of the intake ring, the second intake hole is in communication with the atomizing chamber through the first intake hole.

Furthermore, the mounting seat further includes a first connecting portion protruding from the lower end surface of the receiving portion. After the first connecting portion extending through the bottom wall of the intake ring, the bottom wall of the intake ring is in close contact with the bottom of the receiving portion.

Furthermore, a first fixing hole is defined at the bottom wall of the mounting portion, and a second fixing hole is defined on the second electrode. One end of the heating member is detachably connected to the mounting portion by the engagement of a first screw and the first fixing hole, the opposite end of the heating member is detachably connected to the second electrode by the engagement of a second screw and the second fixing hole.

An electronic cigarette includes any one of the above-mentioned atomizers.

The beneficial effects of the atomizer or the electronic cigarette of the present disclosure are as follows:
(1) The liquid absorbing member and the heating member received in the atomizing chamber are detachably mounted on the bottom wall of the mounting portion, thereby realizing the function of quickly changing the liquid absorbing member and the heating member, and having a simple structure and convenient operation.
(2) The liquid storage assembly is provided with the liquid storage chamber in communication with the atomizing chamber, which can store the cigarette liquid, thereby avoiding the trouble of frequently adding the cigarette liquid during the user's use.

Another technical solution of the present disclosure which is adopted to solve the technical problems is that: an atomizer includes a housing, a base assembly detachably mounted at the lower end of the housing and an atomizing assembly received in the housing, the atomizing assembly includes a liquid absorbing member and at least two heating members, the at least two heating members are disposed on the base assembly, at least one mounting groove is formed between the at least two heating members, the liquid absorbing member is detachably inserted in the at least one mounting groove.

Furthermore, the base assembly includes a mounting seat detachably received in the bottom of the housing, a space between the upper surface of the mounting seat and the inner wall of the housing forms an atomizing chamber, the number of the heating member is two, the two heating members are both in the form of a sheet, the two heating members are oppositely detachably disposed at the upper surface of the mounting seat.

Furthermore, the base assembly further includes a second electrode and a third electrode disposed on the upper surface of the mounting seat, the second electrode and the third electrode are oppositely disposed, and the second electrode and the third electrode are sandwiched between the two heating members, the second electrode and the third electrode are detachably connected to corresponding ends of the two heating members, respectively, the two heating members, the second electrode and the third electrode are collectively enclosed to form the mounting groove.

Furthermore, the second electrode is provided with at least one second mounting hole, the third electrode is provided with at least one third mounting hole, the at least one second mounting hole and the at least one third mounting hole are detachably coupled to the corresponding ends of the two heating members by fasteners, respectively.

Furthermore, the base assembly further includes a first electrode, a first insulating member and a second insulating member, a second mounting tube extends downward from the bottom surface of the mounting seat and is in communication with the inner cavity of the mounting seat, the first electrode is mounted in the second mounting tube, and the first electrode is insulated from the mounting seat by the first insulating member, the second electrode is electrically connected with the first electrode, and the second electrode is insulated from the mounting seat by the second insulating member, the third electrode is electrically connected to the mounting seat.

Furthermore, the second electrode has a "L"-shaped structure, the horizontal edge of the second electrode is detachably sleeved on the upper end of the first electrode and electrically connected to the first electrode, the third electrode is disposed on the upper surface of the mounting seat with respect to the longitudinal edge of the second electrode.

Furthermore, the side wall of the mounting seat is provided with at least one fourth intake hole in communication with the atomizing chamber, a third intake hole corresponding to the at least one fourth intake hole(s) is defined in the sidewall of the lower portion of the housing, the third intake hole(s) are respectively in communication with the outside and the corresponding fourth intake hole(s).

Furthermore, the atomizer further includes a mouthpiece connector, the mouthpiece connector is detachably mounted at the upper end of the housing, the internal space of the mouthpiece connector forms an air outlet passage, the air outlet passage is in communication with the atomizing chamber.

Furthermore, the upper end of the housing extends inward in the radial direction of the housing to form an abutting portion, the end of the abutting portion extends downward in the axial direction of the housing to form a second connecting portion, the lower portion of the mouthpiece connector is necked to form a mounting portion, the constricted neck portion of the mouthpiece connector forms a constricted neck end surface, the mounting portion is detachably coupled to the second connecting portion, the abutting portion abuts against the constricted neck end surface.

An electronic cigarette includes any one of the above-mentioned atomizers.

The beneficial effects of the atomizer or the electronic cigarette of the present disclosure are as follows: the liquid absorbing member is detachably inserted into the mounting groove formed by the heating members, thereby realizing quick replacement of the liquid absorbing member, simple structure and convenient operation.

Still another technical solution of the present disclosure which is adopted to solve the technical problems is that: an atomizing assembly for mounting on an atomizer of an electronic cigarette, the atomizing assembly includes a mounting seat, a liquid absorbing member, a heating member and a fixing member, the mounting seat includes a main body portion, and a second electrode and a third electrode spaced apart from each other and protruded on the same surface of the main body portion, both the second electrode and the third electrode are detachably connected to the fixing member after passing through the liquid absorbing member and the heating member in sequence.

Furthermore, the second electrode and the third electrode are spaced apart from each other and protruded on the top surface of the main body portion, respectively, the liquid absorbing member includes a first lower surface in contact with the top surface of the main body portion, a first upper surface opposite to the first lower surface, and two receiving holes extending through the first lower surface and the first upper surface, the heating member includes a second lower surface in contact with the first upper surface, a second upper surface opposite to the second lower surface, and two through holes extending through the second lower surface and the second upper surface, both the second electrode and the third electrode are detachably connected to the fixing member after sequentially passing through the corresponding receiving holes and the through holes.

Furthermore, the mounting seat further includes a second liquid inlet groove, the second liquid inlet groove extends through the top surface and the bottom surface of the main body portion, and the second liquid inlet groove is in communication with the liquid absorbing member.

Furthermore, the second electrode and the third electrode are disposed along the periphery of the main body portion, and the third electrode and the second electrode are symmetrically disposed along the radial direction of the main body portion, the second liquid inlet groove is provided in the direction perpendicular to the line connecting the second electrode and the third electrode.

Furthermore, the mounting seat further includes a first electrode and a first insulating member, the third electrode is electrically connected to the main body portion, the inner side of one end of the second electrode toward the main body portion is provided with an accommodating cavity, one end of the first electrode is received in the accommodating cavity after passing through the top surface of the main body portion and electrically connected to the second electrode, the opposite end of the first electrode passes through the main body portion and protrudes outside the bottom surface of the main body portion, the first insulating member is disposed between the main body portion and the first electrode.

Furthermore, the heating member is provided with at least one smoke outlet groove for emitting smoke.

Furthermore, the number of the fixing member is two, the second electrode and the third electrode are respectively detachably connected to the corresponding fixing member by threads.

Furthermore, the heating member is a stainless steel heating sheet.

Furthermore, the atomizing assembly further includes a sleeve, the sleeve is provided with a receiving cavity, the mounting seat, the liquid absorbing member and the heating member are all received in the receiving cavity.

An atomizer includes any one of the above-mentioned atomizing assembly.

An electronic cigarette includes the above-mentioned atomizer.

Compared with the prior art, the atomizing head, the atomizer and the electronic cigarette of the present disclosure have at least the following advantages: the atomizing head includes a negative electrode seat, a third electrode disposed on the negative electrode seat and a second electrode coupled to the negative electrode seat, the liquid absorbing member is disposed on the negative electrode seat through the receiving holes, the heating member is disposed on the negative electrode seat through the through holes and is disposed on the liquid absorbing member. The liquid absorbing member and the heating member are screwed to the third electrode and the second electrode through the fixing member, and can be removed and replaced, which is convenient to use and simple to operate.

### BRIEF DESCRIPTION OF THE DRAWINGS

The present disclosure will be further described withe reference to the accompanying drawings and embodiments.
FIG. 1 is a perspective view of an atomizer according to a first embodiment of the present disclosure;
FIG. 2 is an exploded view of the atomizer shown in FIG. 1;
FIG. 3 is a schematic view of an atomizing assembly of the atomizer shown in FIG. 2;
FIG. 4 is another schematic view of the atomizing assembly of the atomizer shown in FIG. 2;
FIG. 5 is a front view of the atomizer shown in FIG. 1;
FIG. 6 is a cross-sectional view of the atomizer of FIG. 5 taken along line A-A;
FIG. 7 is another cross-sectional view of the atomizer shown in FIG. 5; (rotating 90° relative to FIG. 6)
FIG. 8 is a cross-sectional view of an atomizer in a second embodiment of the present disclosure.
FIG. 9 is a schematic view of an atomizer according to a third embodiment of the present disclosure;
FIG. 10 is an exploded view of the atomizer shown in FIG. 9;
FIG. 11 is a cross-sectional view of the atomizer of FIG. 9 taken along line B-B;
FIG. 12 is another cross-sectional view of the atomizer shown in FIG. 9 (rotated 90 ° relative to FIG. 11);
FIG. 13 is a cross-sectional view of an atomizer of a fourth embodiment of the present disclosure;
FIG. 14 is another cross-sectional view of the atomizer shown in FIG. 13 (rotated 90° relative to FIG. 13).
FIG. 15 is a schematic view of an atomizing head according to a fifth embodiment of the present disclosure;
FIG. 16 is a cross-sectional view taken along line C-C of FIG. 15;
FIG. 17 is an exploded view of the atomizing head of FIG. 15.

The component names and reference numerals thereof are as follows:

| | | |
|---|---|---|
| Atomizer 100 | Atomizing assembly 10 | Intake ring 20 |
| Liquid storage assembly 30 | Mounting seat 11 | Internal thread 3023 |
| Receiving portion 111 | Air outlet portion 112 | First connecting portion 113 |
| Liquid absorbing member 12 | Heating member 13 | First electrode 141 |
| First insulating member 142 | Second electrode 143 | Second insulating member 144 |
| Second intake hole 201 | Atomizing chamber 114 | Reservoir tube 301 |
| Top cover 302 | Sealing member 303 | Liquid storage chamber 304 |
| Mounting portion 1111 | First liquid inlet groove 1112 | Conducting mechanism 14 |
| Liquid inlet hole 1114 | First intake hole 1115 | Flange 1116 |
| Stationary ring 1117 | Air outlet passage 1121 | First external thread 1122 |
| First mounting hole 1131 | Second external thread 1132 | Communication tube 3021 |
| First mounting tube 3022 | First fixing hole 1118 | First screw 131 |
| Second fixing hole 1431 | Second screw 132 | Smoke outlet groove 133 |
| Bottom wall 1119 | | |
| Atomizer 200 | Housing 40 | Mouthpiece connector 50 |
| Base assembly 60 | Atomizing assembly 70 | Atomizing chamber 41 |
| Mounting seat 61 | First electrode 62 | First insulating member 63 |
| Second electrode 64 | Second insulating member 65 | Third electrode 66 |
| Heating member 71 | Liquid absorbing member 72 | Abutting portion 401 |
| Second connecting portion 402 | Third intake hole 403 | Mounting end 501 |
| Constricted neck end surface 502 | Air outlet passage 503 | Fourth intake hole 611 |
| Second mounting tube 612 | Horizontal edge 64a | Longitudinal side 64b |
| Second mounting hole 641 | Third mounting hole 661 | Third fixing hole 711 |
| Fourth fixing hole 712 | Smoke outlet groove 713 | Mounting groove 714 |
| Socket 621 | | |
| Negative electrode seat 81 | Main body portion 810 | Second electrode 811 |
| First insulating member 812 | First electrode 813 | Third electrode 814 |
| Second liquid inlet groove 815 | Top surface 816 | Bottom surface 817 |
| Liquid absorbing member 82 | Receiving hole 820 | First lower surface 821 |
| First upper surface 822 | Heating member 83 | Second lower surface 830 |
| Second upper surface 831 | Through hole 832 | Smoke outlet 833 |
| Fixing member 84 | Sleeve 85 | Receiving chamber 850 |
| Mounting seat 86 | Accommodating cavity 818 | |

### DETAILED DESCRIPTION OF EMBODIMENTS

The present disclosure will be described in detail in conjunction with the accompanying drawings. The drawings are simplified schematic diagrams, only for explaining the basic structure of the present disclosure in a schematic manner, therefore only the configuration related to the present disclosure is shown.

### The first embodiment

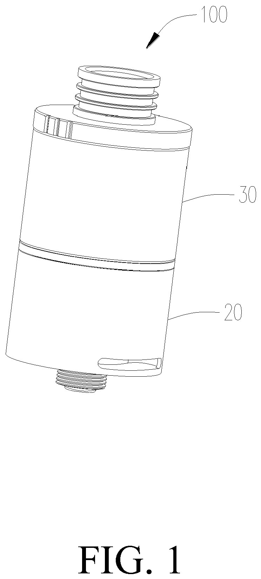

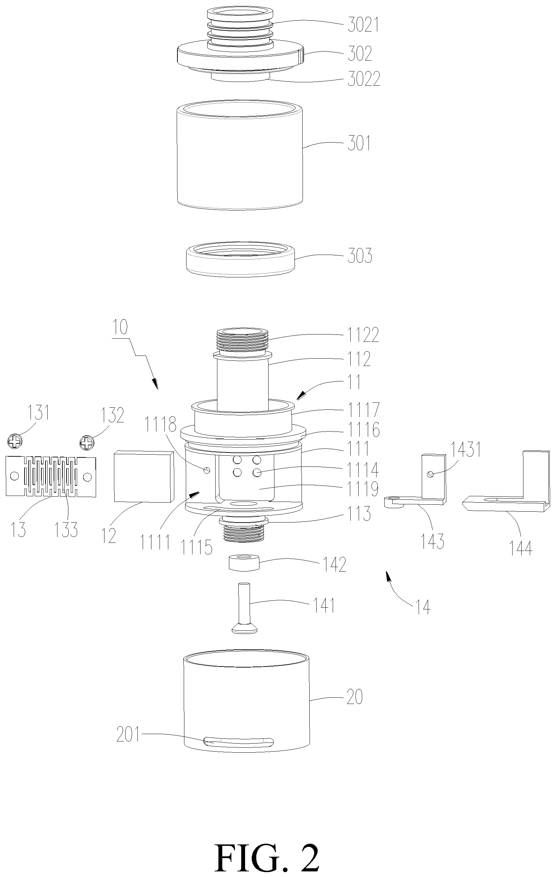

Referring to FIG. 1, FIG. 2 and FIG. 5, the present disclosure provides an electronic cigarette comprising an atomizer 100 and a power supply device (not shown) mounted at a lower end of the atomizer 100. The atomizer 100 includes an atomizing assembly 10, an intake ring 20 sleeved in a lower portion of the atomizing assembly 10, and a liquid storage assembly 30 sleeved on an upper portion of the atomizing assembly 10.

The atomizing assembly 10 includes a mounting seat 11, a liquid absorbing member 12 and a heating member 13 detachably mounted on the mounting seat 11, and a conducting mechanism 14 provided on the mounting seat 11.

The mounting seat 11 includes a receiving portion 111, an air outlet portion 112 protruding from the upper end of the receiving portion 111, and a first connecting portion 113 protruding from the lower end of the receiving portion 111. In the embodiment of the present disclosure, the receiving portion 111, the air outlet portion 112 and the first connecting portion 113 are integrally formed.

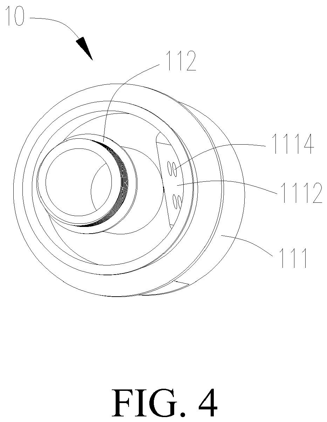

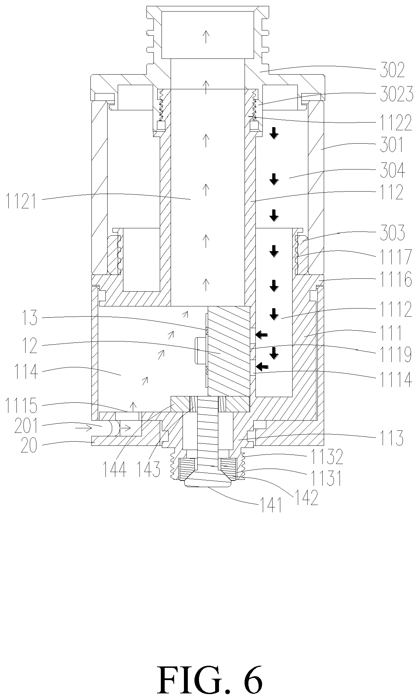

Please refer to FIG. 4 and FIG. 6 at the same time, the receiving portion 111 has a substantially cylindrical structure. A first liquid inlet groove 1112 penetrating the upper end surface of the receiving portion 111 is axially defined in the receiving portion 111. A mounting portion 1111 is recessed inwardly on the sidewall of the receiving portion 111. A liquid inlet hole 1114 is defined at the bottom wall 1119 of the mounting portion 1111. The mounting portion 1111 and the first liquid inlet groove 1112 are in communication via the liquid inlet hole 1114.

The lower end surface of the receiving portion 111 is provided with a first intake hole 1115 that in communication with the mounting portion 1111. A circumferential side of the upper end surface of the receiving portion 111 has a flange 1116 extending outward in the circumferential direction of the receiving portion 111. The upper end surface of the receiving portion 111 further has a stationary ring 1117 protruding upward in the axial direction of the receiving portion 111. The stationary ring 1117 is substantially annular, the stationary ring 1117 is disposed coaxially with the receiving portion 111.

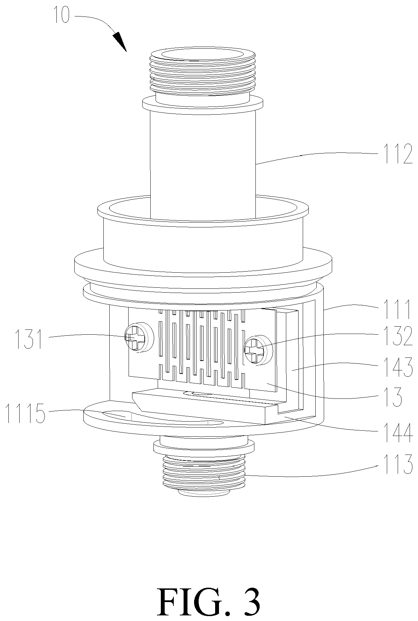

Please refer to FIG. 3 at the same time, the air outlet portion 112 has a substantially cylindrical structure, the air outlet portion 112 is provided at the center of the upper end surface of the receiving portion 111. The air outlet portion 112, the stationary ring 1117 and the receiving portion 111 are coaxially disposed, and the outer diameter of the air outlet portion 112 is smaller than the inner diameter of the stationary ring 1117. The outer diameter of the stationary ring 1117 is less than or equal to the diameter of the receiving portion 111. An air outlet passage 1121 in communication with the mounting portion 1111 is defined in the air outlet portion 112 along the axial direction of the air outlet portion 112. A first external thread 1122 is defined at one end of the air outlet portion 112 away from the receiving portion 111. The first liquid inlet groove 1112 extends through an upper end surface of the receiving portion 111 located between the air outlet portion 112 and the stationary ring 1117.

The first connecting portion 113 has a substantially cylindrical structure. The first connecting portion 113 is disposed at the center of the lower end surface of the receiving portion 111, a first mounting hole 1131 in communication with the mounting portion 1111 is defined in an inner portion of the first connecting portion 113 along the axial direction of the first connecting portion 113, a second external thread 1132 is defined at one end of the first connecting portion 113 away from the receiving portion 111. The atomizer 100 is coupled to the power supply device of the electronic cigarette by the second external thread 1132.

The liquid absorbing member 12 has a substantially flat structure, the liquid absorbing member 12 is disposed in the mounting portion 1111, and an end surface of the liquid absorbing member 12 abuts against the bottom wall 1119 of the mounting portion 1111, the liquid absorbing member 12 is in communication with the liquid inlet hole 1114.

In the embodiment of the present discourse, the material of the liquid absorbing member 12 is porous ceramic. It can be understood that, in other embodiments, the liquid absorbing member 12 can be also made of other porous materials, such as metal foam, foamed graphite, cotton, cotton cloth or sponge.

The heating member 13 has a substantially sheet-like structure, the heating member 13 is in close contact with the end surface of the liquid absorbing member 12 away from the bottom wall 1119 of the mounting portion 1111, and one end of the heating member 13 is detachably fixedly coupled to the mounting portion 1111. The connection method in this embodiment is a screw connection. Specifically, a first fixing hole 1118 is defined at the bottom wall 1119 of the mounting portion 1111, one end of the heating member 13 is detachably fixedly connected to the mounting portion 1111 by the engagement of the first screw 131 and the first fixing hole 1118.

It can be understood that, in other embodiments, it may be connected by a buckle, a latching slot or the like.

In the embodiment of the present disclosure, the heating member 13 is a stainless steel heating sheet. In order to make the smoke generated by heating member 13 heating the cigarette liquid can smoothly flow out from the surface where the heating member 13 and the liquid absorbing member 12 are attached, at least one smoke outlet groove 133 is defined in the heating member 13.

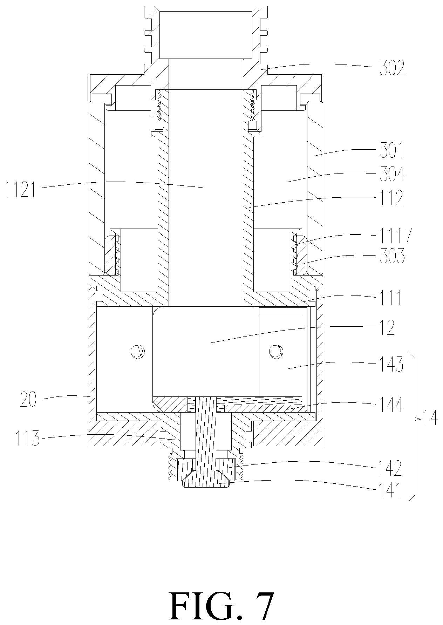

Please refer to FIG. 7 at the same time, the conducting mechanism 14 includes a first electrode 141, a first insulating member 142, a second electrode 143 and a second insulating member 144.

The first electrode 141 is mounted in the first mounting hole 1131 of the first connecting portion 113, and one end of the first electrode 141 is electrically connected to the power supply device.

The first insulating member 142 is fixedly sleeved on the first electrode 141, the outer peripheral surface of the first insulating member 142 is closely connected to the inner peripheral surface of the first mounting hole 1131 of the first connecting portion 113, thereby insulating the first electrode 141 from the first connecting portion 113.

The second electrode 143 is substantially in an inverted "L"-shaped structure, the second electrode 143 is mounted in the mounting portion 1111. One edge of the second electrode 143 is fixedly sleeved at one end of the first electrode 141 opposite to the power supply device, and the second electrode 143 is electrically connected to one end of the first electrode 141 opposite to the power supply device. The other end of the heating member 13 is detachably fixedly connected to the other edge of the second electrode 143. The connection method in this embodiment is a screw connection. Specifically, a second fixing hole 1431 is defined in an edge of the second electrode 143 away from the first electrode 141. The other end of the heating member 13 is detachably fixedly coupled to the other edge of the second electrode 143 by the engagement of the second screw 132 and the second fixing hole 1431.

It can be understood that, in other embodiments, it may be connected by a buckle, a latching slot or the like.

The second insulating member 144 is substantially in an inverted "L"-shaped, the second insulating member 144 is disposed between the second electrode 143 and the receiving portion 111 to insulate the second electrode 143 from the receiving portion 111.

In the embodiment of the present disclosure, the first insulating member 142 and the second insulating member 144 are made of rubber material. It can be understood that, the first insulating member 142 and the second insulating member 144 can also be made of silicone material.

When the power supply device is connected to the first connection portion 113, the positive electrode of the power supply device is electrically connected to the heating member 13 via the first electrode 141 and the second electrode 143, then the heating member 13 is electrically connected to the negative electrode of the power supply device via the receiving portion 111 and the first connecting portion 113, thereby, the circuit is turned on, the heating member 13 is heated to atomize the cigarette liquid under electric driving.

The intake ring 20 has a sleeve-like structure having an opening at the top end, the intake ring 20 is detachably sleeved on the receiving portion 111, and the top end of the intake ring 20 abuts against the lower end surface of the flange 1116. The space between the groove wall of the mounting portion 1111 and the inner wall of the intake ring 20 forms an atomizing chamber 114, at this time, the liquid absorbing member 12 and the heating member 13 are housed in the atomizing chamber 114. After the first connecting portion 113 extends through the bottom wall of the intake ring 20, the bottom wall of the intake ring 20 is in close contact with the bottom of the receiving portion 111.

A second intake hole 201 is defined at the intake ring 20. In this embodiment, the second intake hole 201 is defined at the outer peripheral surface of the bottom wall of the intake ring 20 and is in communication with the first intake hole 1115. Now, the second intake hole 201, the first intake hole 1115, the atomizing chamber 114 and the air outlet passage 1121 are in communication with each other.

The liquid storage assembly 30 includes a reservoir tube 301 sleeved on the stationary ring 1117 and a top cover 302 detachably connected to an end of the air outlet portion 112 away from the receiving portion 111.

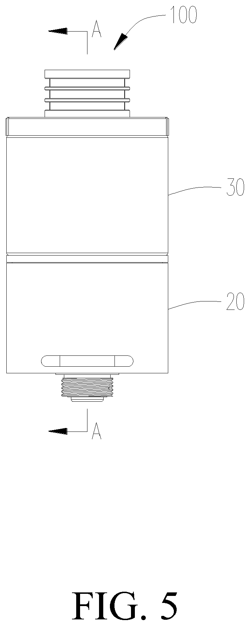

The reservoir tube 301 is substantially a hollow tubular structure having openings at both ends, one end of the reservoir tube 301 is sleeved on the stationary ring 1117 of the receiving portion 111 and supported on the upper end surface of the flange 1116. Referring to FIG. 5 and FIG. 6 , in one embodiment, the liquid storage assembly 30 further includes a sealing member 303, the sealing member 303 is disposed between the outer peripheral surface of the stationary ring 1117 and the inner peripheral surface of the reservoir tube 301, thereby, the reservoir tube 301 is fixedly connected to the receiving portion 111.

The top cover 302 has a substantially disk-like structure, the center of the top surface of top cover 302 extends upwardly to form a communication tube 3021 in communication with the inner cavity of the top cover 302, a first mounting tube 3022 in communication with the communication tube 3021 extends downward at the center of the top surface of the top cover 302, the first mounting tube 3022 is provided with an internal thread 3023 that cooperates with the first external thread 1122 of the air outlet portion 112. After the first external thread 1122 is connected with the internal thread 3023, the communication tube 3021 is in communication with the air outlet passage 1121, the edge of the top cover 302 is supported at one end of the reservoir tube 301 opposite to the receiving portion 111.

In the embodiment of the present disclosure, the communication tube 3021 of the top cover 302 can be directly used as a cigarette holder, the user directly sucks through the communication tube 3021. It can be understood that, in other embodiments, a communication member in communication with the communication tube 3021 is connected to the communication tube 3021, the user sucks through the communication tube 3021.

In the embodiment of the present disclosure, when the user inhales the electronic cigarette, the external air enters the atomizing chamber 114 through the second intake hole 201 and the first intake hole 1115 sequentially, and then mixed with the smoke generated in the atomizing chamber 114 and enters the air outlet passage 1121, and finally flows into the user's mouth through the communication tube 3021. The direction indicated by the thinner arrow in Fig. 6 is the direction of the intake air.

It can be understood that, in other embodiments, the first intake hole 1115 at the bottom of the receiving portion 111 may be omitted. At this time, the second intake hole 201 of the intake ring 20 is defined at the side wall of the intake ring 20, the second intake hole 201 is directly in communication with the atomizing chamber 114.

A space between the inner wall of the reservoir tube 301 and the outer wall of the air outlet portion 112 and a space between the inner wall of the stationary ring 1117 and the outer wall of the air outlet portion 112 form a liquid storage chamber 304, thereby, the first liquid inlet groove 1112 is in communication with the liquid storage chamber 304. When it is necessary to add the cigarette liquid, the user only needs to remove the top cover 302 to inject the cigarette liquid into the liquid storage chamber 304. In use, the cigarette liquid flows into the first liquid inlet groove 1112 in communication with the liquid storage chamber 304, the cigarette liquid in the first liquid inlet groove 1112 is absorbed by the liquid absorbing member 12 through the liquid inlet hole 1114, and then, the cigarette liquid is atomized into smoke by the heating member 13, and is mixed with the air and inhaled by the user. The direction indicated by the thicker arrow in Fig. 6 is the flow direction of the cigarette liquid.

The present disclosure provides an atomizer 100, when the liquid absorbing member 12 and the heating member 13 need to be replaced, the intake ring 20 is pulled out vigorously, thus the liquid absorbing member 12 and the heating member 13 are exposed to the outside, and then the screw is removed, the liquid absorbing member 12 and the heating member 13 can be removed from the receiving portion 111. During installation, the new liquid absorbing member 12 and the heating member 13 are replaced and fastened by screws, and finally the intake ring 20 is sleeved on the receiving portion 111. Compared with the conventional atomizer structure, the purpose of quickly changing the liquid absorbing member 12 and the heating member 13 can be achieved, the structure is simple and the operation is convenient. In addition, the atomizer 100 of the present disclosure further has a liquid storage chamber 304, which avoids the trouble of the user frequently adding the cigarette liquid, and greatly improves the user experience.

The present disclosure provides an electronic cigarette having all the technical features of the atomizer 100 described above, thus the electronic cigarette has the same technical effects as the atomizer 100 described above.

### The second embodiment

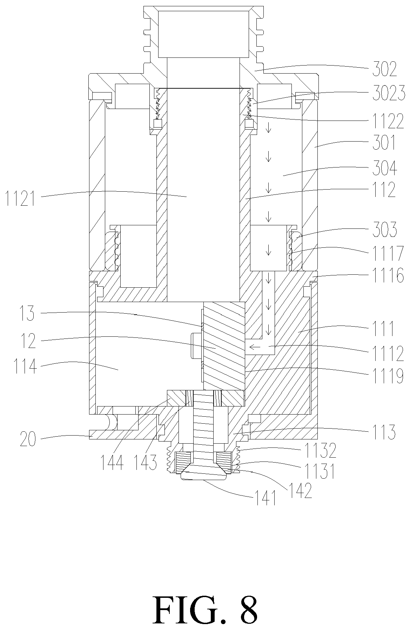

Referring to FIG. 8, the difference between the atomizer of the second embodiment and the atomizer 100 of the first embodiment is as follows: in the second embodiment, the liquid inlet hole 1114 on the bottom wall 1119 of the mounting portion 1111 is omitted, the first liquid inlet groove 1112 is directly in communication with the liquid absorbing member 12. Specifically, the first liquid inlet groove 1112 includes a first section and a second section, the first section of the first liquid inlet groove 1112 is defined in the axial direction of the receiving portion 111, the second section of the first liquid inlet groove 1112 is defined in the radial direction of the receiving portion 111. One end of the first section of the first liquid inlet groove 1112 is in communication with one end of the second section of the first liquid inlet groove 1112, and the other end of the first section of the first liquid inlet groove 1112 extends through the upper end surface of the receiving portion 111 and is in communication with the liquid storage chamber 304, the other end of the second section of the first liquid inlet groove 1112 extends through the bottom wall 1119 of the mounting portion 1111 and communicates with the liquid absorbing member 12 .

In use, the cigarette liquid injected into the liquid storage chamber 304 is directly absorbed by the liquid absorbing member 12 via the first liquid inlet groove 1112, and is heated and atomized by the heating member 13. The direction indicated by the arrow in Fig. 8 is the flow direction of the cigarette liquid. Compared with the atomizer 100 of the first embodiment, the atomizer 100 of the second embodiment is simpler in structure and easier to process.

### The third embodiment

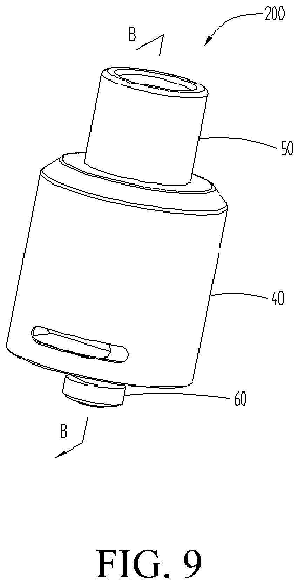

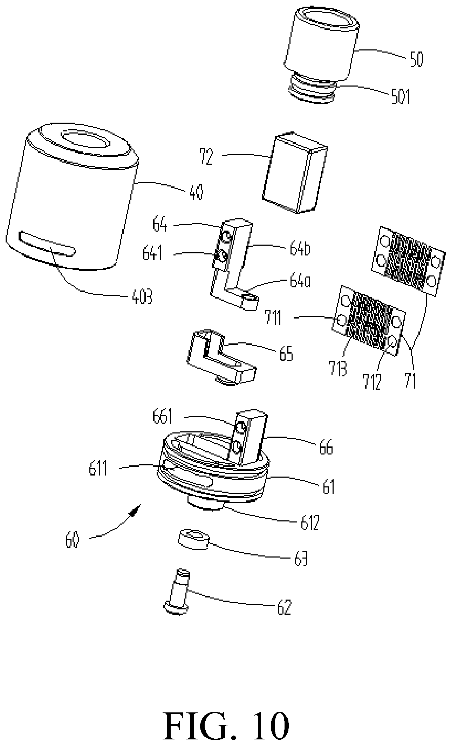

Referring to FIGS. 9-11, the present disclosure provides an electronic cigarette including an atomizer 200 and a power supply device (not shown) mounted at a lower end of the atomizer 200. The atomizer 200 includes a housing 40, a mouthpiece connector 50 detachably mounted to the upper end of the housing 40, a base assembly 60 detachably mounted to the lower end of the housing 40, and an atomizing assembly 70 received within the housing 40.

The housing 40 is substantially a sleeve-like structure having openings at both ends, an upper end of the housing 40 extends inward in the radial direction of the housing 40 to form an abutting portion 401, the end of the abutting portion 401 extends downward in the axial direction of the housing 40 to form a second connecting portion 402 having a tubular structure.

Two third intake holes 403 are oppositely disposed on the sidewall of the lower portion of the housing 40, the third intake hole 403 is in communication with the inner cavity of the housing 40. It can be understood that, the third intake hole(s) 403 can also be one or more than two.

The mouthpiece connector 50 is substantially a cylindrical structure having openings at both ends, the lower portion of the mouthpiece connector 50 is necked to form a mounting end 501, the constricted neck portion of the mouthpiece connector 50 forms a constricted neck end surface 502. The mounting end 501 is detachably coupled to the second connecting portion 402 of the housing 40 to enable the mouthpiece connector 50 to communicate with the inner cavity of the housing 40. The abutting portion 401 of the housing 40 abuts against the constricted neck end surface 502 of the mouthpiece connector 50.

It can be understood that, the connection manner of the mounting end 501 and the second connecting portion 402 can be connected by a threaded fit, or can be connected by closely fitting the mounting end 501 to the second connecting portion 702. In the embodiment, the mounting end 501 is connected to the second connecting portion 702 by means of plugging, and is easy to assemble and disassemble, and is convenient to use.

The internal space of the mouthpiece connector 50 forms an air outlet passage 503.

In the embodiment of the present disclosure, the mouthpiece connector 50 can be directly used as a mouthpiece, the user directly inhales through the mouthpiece connector 50. It can be understood that, in other embodiments, a communication member in communication with the mouthpiece connector 50 can also be connected to the mouthpiece connector 50 through which the user inhales.

The base assembly 60 includes a mounting seat 61 and a first electrode 62, a first insulating member 63, a second electrode 64, a second insulating member 65, and a third electrode 66 respectively mounted on the mounting seat 61.

The mounting seat 61 has a substantially disk-like structure, the mounting seat 61 is detachably received at the bottom of the housing 40, the space between the upper surface of the mounting seat 61 and the inner wall of the housing 40 forms an atomizing chamber 41, the atomizing chamber 41 is in communication with the air outlet passage 503 of the mouthpiece connector 50, a fourth intake hole 611 communicating with the atomizing chamber 41 is defined at the side wall of the mounting seat 61 corresponding to the third intake hole 403.

A second mounting tube 612 extends downward at the center of the bottom surface of the mounting seat 61 and is in communication with the inner cavity of the mounting seat 61, the first electrode 62 is mounted in the second mounting tube 612 through the first insulating member 63, so that the first electrode 62 is insulated from the mounting seat 61, the lower end of the first electrode 62 is electrically connected to the power supply device.

In the embodiment of the present disclosure, the first insulating member 63 is made of a rubber material. It can be understood that, the first insulating member 63 can also be made of a silicone material to achieve insulation.

The second electrode 64 has a substantially "L"-shaped structure, the second electrode 64 is mounted on the upper surface of the mounting seat 61, the horizontal edge 64a of the second electrode 64 is detachably sleeved on the upper end of the first electrode 62 and electrically connected to the first electrode 62. At least one second mounting hole 641 is defined in the longitudinal edge 64b of the second electrode 64. In the embodiment of the present disclosure, there are two second mounting holes 641.

The second insulating member 65 has a substantially "L"-shaped structure, the second insulating member 65 is disposed between the mounting seat 61 and the second electrode 64, thereby insulating the second electrode 64 from the mounting seat 61.

In the embodiment of the present disclosure, the second insulating member 65 is made of rubber material. It can be understood that, the second insulating member 65 can also be made of silicone material to achieve insulation.

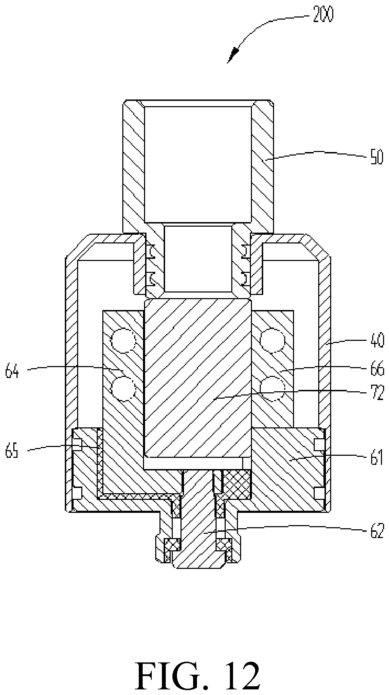

Referring to FIG. 12 at the same time, the third electrode 66 is fixedly disposed on the upper surface of the mounting seat 61 with respect to the longitudinal edge 64b of the second electrode 64, the third electrode 66 is provided with at least one third mounting hole 661. In the embodiment of the present disclosure, there are two third mounting holes 661. It can be understood that, the third electrode 66 can also be formed by being protruded from the upper surface of the mounting seat 61.

The atomizing assembly 70 includes a heating member 71 and a liquid absorbing member 72.

The heating member 71 has a substantially sheet structure, one end of the heating member 71 is provided with a third fixing hole 711 corresponding to the second mounting hole 641, one end of the heating member 71 opposite to the third fixing hole 711 has a fourth fixing hole 712 corresponding to the third mounting hole 661. One end of the heating member 71 is detachably mounted on the second electrode 64 via a third fixing hole 711 and a second mounting hole 641 by a fastener (not shown). The other end of the heating member 71 is detachably mounted to the third electrode 66 via the fourth fixing hole 712 and the third mounting hole 661 by a fastener. In the present embodiment, the fastener described above is a screw.

It can be understood that, in other embodiments, the heating member 71 can be detachably mounted on the second electrode 64 and the third electrode 66 by means of a buckle, a snap slot or the like.

In the embodiment of the present disclosure, the heating member 71 is a stainless steel heating sheet. In order to make the smoke generated by the heating member 71 heating the cigarette liquid fill the atomizing chamber, facilitating the full mixing of the smoke and the air, the heating member 71 is provided with at least one smoke outlet groove 713.

In the embodiment of the present disclosure, there are two heating members 71 oppositely disposed, the second electrode 64 and the third electrode 66 are also disposed opposite to each other and sandwiched between the two heating members 71. The cavity formed by the enclosing of the two heating members 71, the second electrode 64 and the third electrode 66 constitutes a mounting groove 714.

The liquid absorbing member 72 has a substantially flat structure, the liquid absorbing member 72 is disposed in the mounting groove 714, and the liquid absorbing member 72 is in close contact with the heating member 71. When it is need to inject cigarette liquid, it is only necessary to remove the mouthpiece connector 50 and drop the cigarette liquid to the liquid absorbing member 72. The liquid absorbing member 72 has an adsorption function and can store a certain amount of cigarette liquid to meet the user's need for smoking during a period of time.

In the embodiment of the present disclosure, the material of the liquid absorbing member 72 is a porous ceramic. It can be understood that, in other embodiments, the liquid absorbing member 72 can also be other porous materials such as metal foam, foamed graphite, cotton, cotton cloth or sponge.

When the power supply device is connected to the atomizer 200 of the present disclosure, the positive electrode of the power supply device is electrically connected to the heating member 71 via the first electrode 62 and the second electrode 64, the heating member 71 is then electrically connected to the negative electrode of the power supply device via the third electrode 66 and the mounting seat 61, thereby, the circuit is turned on, the heating member 71 is heated to atomize the cigarette liquid under electric driving.

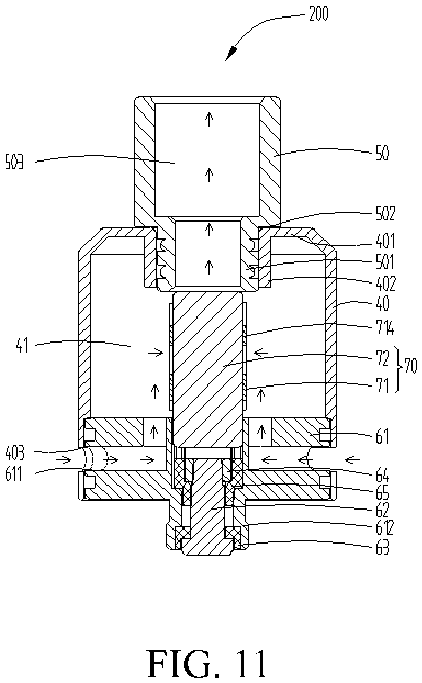

When the user smokes the electronic cigarette, the external air enters into the atomizing chamber 41 via the third intake hole 403 on the housing 40 and the fourth intake hole 611 on the mounting seat 61, sequentially, and enters the outlet passage 503 of the mouthpiece connector 50 after mixing with the generated smoke in the atomizing chamber 41, and finally enters the user's mouth. Specifically, the direction indicated by the arrow in FIG. 11 is the flow direction of the air.

The present disclosure provides an atomizer 200, when the heating member 71 and the liquid absorbing member 72 need to be replaced, the housing 40 is removed, thus the heating member 71 and the liquid absorbing member 72 are exposed to the outside, and the old liquid absorbing member 72 is directly taken out from the mounting groove 714, remove the screw and remove the heating member 71; during installation, a new heating member 71 is replaced and fastened by screws, a new liquid absorbing member 72 is inserted into the mounting groove 714, and finally the housing 40 is fitted to the mounting seat 61. Compared with the conventional atomizer structure, the atomizer 200 of the present disclosure can achieve the purpose of quickly replacing the heating member 71 and the liquid absorbing member 72, and has a simple structure and convenient operation.

The present disclosure provides an electronic cigarette having all the technical features of the atomizer 200 described above, thus the electronic cigarette has the same technical effects as the atomizer 200 described above.

### The fourth embodiment

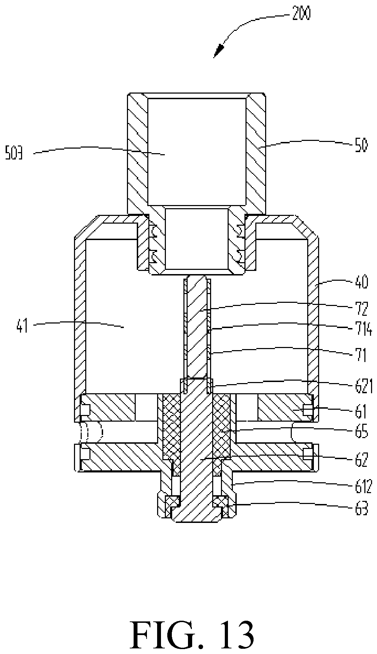

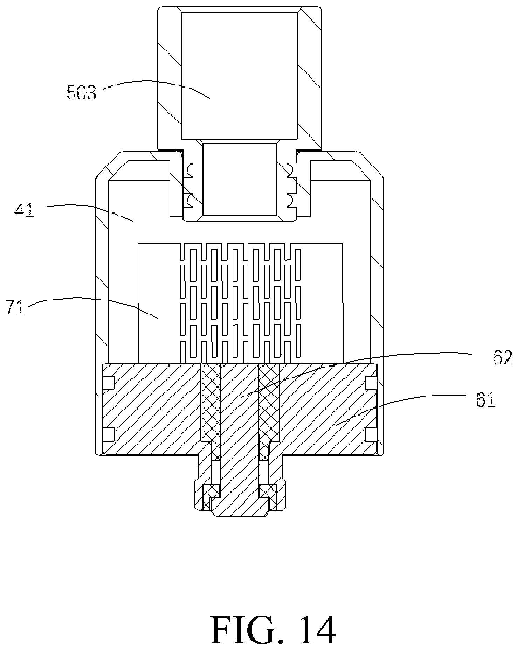

Referring to FIG. 13, the main difference between the atomizer 200 of the fourth embodiment of the present disclosure and the atomizer 200 of the third embodiment is that the second electrode 64, the third electrode 66, the third fixing hole 711 and the fourth fixing hole 712 are omitted. A pair of opposite sockets 621 are defined in the upper surface of one end of the first electrode 62 adjacent to the heating member 71. Two heating members 71 are relatively closely inserted into the sockets 621. Specifically, the groove bottom of the socket 621 is flush with or lower than the upper surface of the mounting seat 61. When the heating members 71 are inserted in the socket 621, as shown in FIG. 14, the lower end of the heating member 71 located on opposite sides of the socket 621 abuts against the upper surface of the mounting seat 61, the liquid absorbing member 72 is directly inserted into the mounting groove 714 formed between the two heating members 71. Thereby, the positive electrode of the power supply device is electrically connected to the heating member 71 via the first electrode 62, the heating member 71 is electrically connected to the negative electrode of the power supply device via the mounting seat 61.

It can be understood that, the heating members 71 may be more than two, it is only necessary to ensure that at least one mounting groove 714 for inserting the liquid absorbing member 72 is formed between the heating members 71.

It can be understood that, when the heating members 71 are more tan two, the arrangement of the heating members 71 is different, the number of the mounting grooves 714 formed between the heating members 71 is also different. For example, when the heating members 71 are arranged around the same axis, there is only one mounting groove 714 formed. For another example, when the heating members 71 are spaced along a straight line, the number of the mounting grooves 714 is one less than the total number of the heating members 71.

When the heating member 71 and the liquid absorbing member 72 need to be replaced, the housing 40 is removed, the heating member 71 and the liquid absorbing member 72 are exposed to the outside, the old liquid absorbing member 72 is directly taken out from the mounting groove 714, and the heating members 71 are pulled out from the socket 621 of the first electrode 62. When installing, the new heating members 71 are inserted into the socket 621, a new liquid absorbing member 72 is inserted into the mounting groove 714, and finally the housing 40 is sleeved on the mounting seat 61. Compared with the atomizer 200 of the third embodiment, the atomizer 200 of the fourth embodiment has a simpler structure, is more convenient to operate, and is easier to process.

### The fifth embodiment

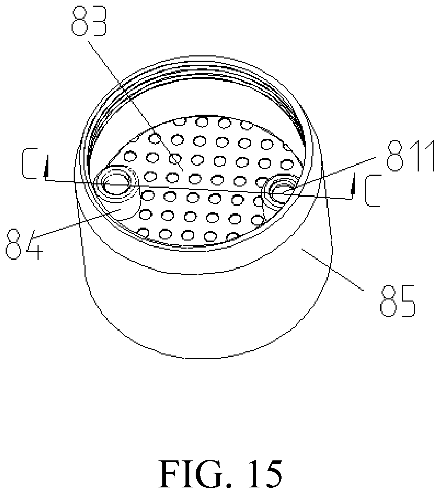

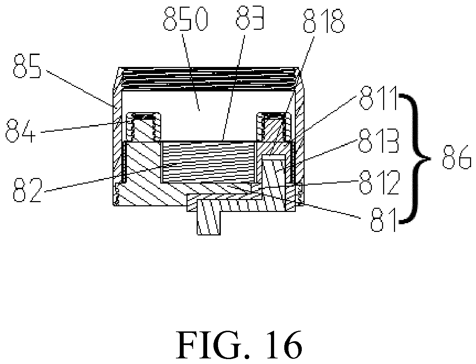

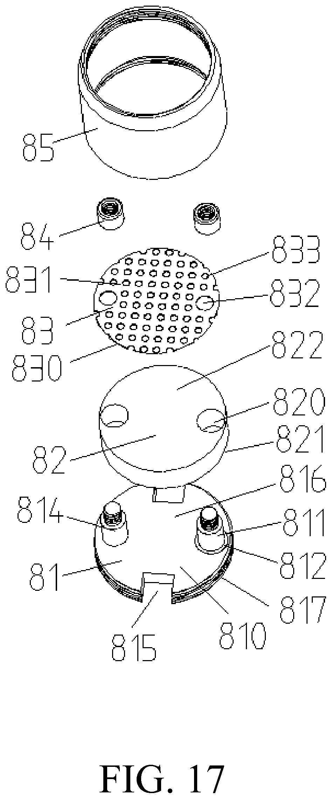

FIGs.15-17 shows an atomizing assembly of the present disclosure, for mounting on an atomizer of an electronic cigarette, including a mounting seat 86, a liquid absorbing member 82 mounted on the mounting seat 86, a heating member 83 stacked on the liquid absorbing member 82, a fixing member 84 that detachably fixes the liquid absorbing member 82 and the heating member 83 to the mounting seat 86 together, and a sleeve 85 connected to the mounting seat 86. The sleeve 85 has a hollow structure and is provided with a receiving cavity 850. The mounting seat 86, the liquid absorbing member 82 and the heating member 83 are all received in the receiving cavity 850. In this embodiment, the fixing member 84 is a screw.

The mounting seat 86 includes a negative electrode seat 81 on which the liquid absorbing member 82 is mounted, a second electrode 811 disposed on the negative electrode seat 81, a first electrode 813 electrically connected to the second electrode 811, and a first insulating member 812 disposed between the first electrode 813 and the negative electrode seat 81 and sleeved on the outer circumference of the first electrode 813. The first insulating member 812 can be made of a silicone rubber or a rubber gasket. The second electrode 811 is a positive electrode connection post, the first electrode 813 is a positive electrode block.

The negative electrode seat 81 includes a main body portion 810, a third electrode 814 and a second liquid inlet groove 815. The third electrode 814 is electrically connected to the main body portion 810. The second electrode 811 and the third electrode 814 are spaced apart from each other and protrudes on the top surface 816 of the main body portion 810, respectively. The second liquid inlet groove 815 extends through the top surface 816 and the bottom surface 817 of the main body portion 810, the second liquid inlet groove 815 is in communication with the liquid absorbing member 82. In this embodiment, the main body portion 810 is substantially disk-shaped. The third electrode 814 is disposed at an angle of 180 degrees with the second electrode 811 in the circumferential direction of the main body portion 810. The second liquid inlet groove 815 is provided in a direction perpendicular to the line connecting the second electrode 811 and the third electrode 814. The third electrode 814 and the second electrode 811 are in step shape of large lower part and small upper part, respectively, and the upper end of both are provided with threads engaging with the fixing member 84. Furthermore, the third electrode 814 and the second electrode 811 are symmetrically disposed along the radial direction of the main body portion 810. Furthermore, the third electrode 814 and the second electrode 811 protrude from the top surface 816 and are disposed along the periphery of the main body portion 810 for better fixing to a better strength. The third electrode 814 is a negative connection post.

The second electrode 811 is provided with an accommodating cavity 818 at the inner side of one end facing the negative electrode seat 81. One end of the first electrode 813 extending through the top surface 816 is received in the accommodating cavity 818 and is in an interference fit with and electrically connected to the second electrode 811. The opposite end of the first electrode 813 passes through the main body portion 810 and protrudes outside the bottom surface 817 of the main body portion 810.

The liquid absorbing member 82 has a cylindrical shape, and includes a first lower surface 821 which can be disposed in contact with the top surface 816, a first upper surface 822 opposite to the first lower surface 821 and two receiving holes 820 extending through the first lower surface 821 and the first upper surface 822. The third electrode 814 and the second electrode 811 are respectively received in one of the receiving holes 820. The two receiving holes 820 are disposed along the circumference of the liquid absorbing member 82 and the centers of the two receiving holes 820 are symmetric with respect to the radial direction of the liquid absorbing member 82.

The heating member 83 includes a second lower surface 830 that can be attached to the first upper surface 822 and a second upper surface 831 opposite to the second lower surface 830. The heating member 83 is a stainless steel heating sheet. The heating member 83 further includes a through hole 832 extending through the second lower surface 830 and the second upper surface 831 and corresponding to the receiving holes 820 of the liquid absorbing member 82, and at least one smoke outlet groove 833 provided on the side of the through hole 832. The third electrode 814 and the second electrode 811 of the negative electrode seat 81 extends through the through hole 832. In one embodiment, the at least one smoke outlet groove 833 are arranged in a matrix on the heating member 83 to ensure uniform smoke emitting. The negative electrode seat 81, the liquid absorbing member 82 and the heating member 83 are disposed from the bottom to the top.

The number of the fixing members 84 is two, the portion of the third electrode 814 which passes through the corresponding receiving hole 820 and the through hole 832 is threaded with one of the fixing members 84, the portion of the second electrode 811 which passes through the corresponding receiving hole 820 and the through hole 832 is threaded with the other fixing member 84, thereby, the liquid absorbing member 82 and the heating member 83 are detachably fixed to the mounting seat 86 together.

The sleeve 85 is a hollow structure having openings at both ends, the mounting seat 86 blocks one of the openings of the sleeve 85, the cigarette liquid flows into the receiving chamber 850 through the second liquid inlet groove 815, and is adsorbed by the liquid absorbing member 82, the heating member 83 heats the cigarette liquid adsorbed by the liquid absorbing member 82 to form smoke under electric driving, the generated smoke flows out through the smoke outlet groove 833 and the other opening of the sleeve 85.

The atomizing assembly of the present disclosure includes a negative electrode seat 81, a third electrode 814 disposed on the negative electrode seat 81 and a second electrode 811 coupled to the negative electrode seat 81. The liquid absorbing member 82 is disposed on the negative electrode seat 81 through the receiving holes 820. The heating member 83 is disposed on the negative electrode seat 81 through the through holes 820 and is located on the liquid absorbing member 82. The first lower surface 821 of the liquid absorbing member 82 is attached to the top surface 816 of the negative electrode seat 81, and the second lower surface 830 of the heating member 83 is attached to the first upper surface 822 of the liquid absorbing member 82. The atomizing assembly is screwed to the third electrode 814 and the second electrode 811 through the fixing members 84, thereby the liquid absorbing member 82 and the heating member 83 are fixed to the negative electrode seat 81, since the liquid absorbing member 82 and the heating member 83 are consumables, thus the liquid absorbing member 82 and the heating member 83 can be removed and replaced, and the atomizing assembly does not need to be replaced as a whole, which is convenient to use and simple to operate.

The present disclosure also provides an atomizer using the above-mentioned atomizing assembly.

The present disclosure also provides an electronic cigarette using the above-mentioned atomizer.

According to the above-described preferred embodiments of the present disclosure, through the above description, various changes and modifications can be made by the relevant workers without departing from the scope of the present disclosure. The technical scope of the present disclosure is not limited to the contents of the specification. The technical scope must be determined according to the scope of the claims.

## Claims

**1.** An atomizer, comprising an atomizing assembly and an intake ring sleeved at a lower portion of the atomizing assembly, wherein the atomizing assembly comprises a mounting seat, a liquid absorbing member and a heating member, the mounting seat comprises a receiving portion, the receiving portion is recessed with a mounting portion, the intake ring is detachably sleeved on the receiving portion, an atomizing chamber is formed between the groove wall of the mounting portion and the inner wall of the intake ring, the liquid absorbing member and the heating member are detachably mounted on the mounting portion.

**2.** The atomizer according to claim 1, wherein a side wall of the receiving portion is recessed inwardly to form the mounting portion, one end surface of the liquid absorbing member is closely attached to the bottom wall of the mounting portion, the heating member is in close contact with the end surface of the liquid absorbing member away from the bottom wall of the mounting portion, the atomizing assembly further comprises a conducting mechanism, the conducting mechanism comprises a second electrode mounted on the mounting portion, one end of the heating member is detachably coupled to the mounting portion, the opposite end of the heating member is detachably coupled to the second electrode.

**3.** The atomizer according to claim 2, wherein the atomizer further comprises a liquid storage assembly sleeved on an upper portion of the atomizing assembly, the liquid storage assembly is provided with a liquid storage chamber therein, the receiving portion is provided with a first liquid inlet groove in communication with the liquid storage chamber and the liquid absorbing member.

**4.** The atomizer according to claim 3, wherein the mounting seat further comprises an air outlet portion protruding from the upper end surface of the receiving portion, the liquid storage assembly comprises a reservoir tube sleeved outside the air outlet portion, the liquid storage chamber is formed by a space between the inner wall of the reservoir tube and the outer wall of the air outlet portion.

**5.** The atomizer according to claim 4, wherein the first liquid inlet groove is defined along the axial direction of the receiving portion, one end of the first liquid inlet groove passes through the upper end surface of the receiving portion and is in communication with the liquid storage chamber, a liquid inlet hole is defined at the bottom wall of the mounting portion, the other end of the first liquid inlet groove is in communication with the liquid absorbing member through the liquid inlet hole; or the first liquid inlet groove comprises a first section and a second section, the first section of the first liquid inlet groove is defined along the axial direction of the receiving portion, the second section of the first liquid inlet groove is defined in the radial direction of the receiving portion, one end of the first section of the first liquid inlet groove is in communication with one end of the second section of the first liquid inlet groove, the opposite end of the first section of the first liquid inlet groove extends through the upper end surface of the receiving section and is in communication with the liquid storage chamber, the opposite end of the second section of the first liquid inlet groove passes through the bottom wall of the mounting portion and is in communication with the liquid absorbing member.

**6.** The atomizer according to claims 4 or 5, wherein an air outlet passage is defined in the air outlet portion along the axial direction of the air outlet portion, a second intake hole is defined at the side wall of the intake ring, both the air outlet passage and the second intake hole are in communication with the atomizing chamber.

**7.** The atomizer according to claim 6, wherein the lower end surface of the receiving portion is provided with a first intake hole, the second intake hole is defined at the outer peripheral surface of the bottom wall of the intake ring, the second intake hole is in communication with the atomizing chamber through the first intake hole.

**8.** The atomizer according to claim 2, wherein the mounting seat further comprises a first connecting portion protruding from the lower end surface of the receiving portion, after the first connecting portion extending through the bottom wall of the intake ring, the bottom wall of the intake ring is in close contact with the bottom of the receiving portion.

**9.** The atomizer according to claim 2, wherein a first fixing hole is defined at the bottom wall of the mounting portion, a second fixing hole is defined on the second electrode, one end of the heating member is detachably connected to the mounting portion by the engagement of a first screw and the first fixing hole, the opposite end of the heating member is detachably connected to the second electrode by the engagement of a second screw and the second fixing hole.

**10.** An electronic cigarette, comprising an atomizer according to any one of claims 1-9.

**11.** An atomizer, comprising a housing, a base assembly detachably mounted at a lower end of the housing and an atomizing assembly received in the housing, wherein the atomizing assembly comprises a liquid absorbing member and at least two heating members, the at least two heating members are disposed on the base assembly, at least one mounting groove is formed between the at least two heating members, the liquid absorbing member is detachably inserted in the at least one mounting groove.

**12.** The atomizer according to claim 11, wherein the base assembly includes a mounting seat detachably received in the bottom of the housing, a space between the upper surface of the mounting seat and the inner wall of the housing forms an atomizing chamber, the number of the heating member is two, the two heating members are both in the form of a sheet, the two heating members are oppositely detachably disposed at the upper surface of the mounting seat.

**13.** The atomizer according to claim 12, wherein the base assembly further includes a second electrode and a third electrode disposed on the upper surface of the mounting seat, the second electrode and the third electrode are oppositely disposed, and the second electrode and the third electrode are sandwiched between the two heating members, the second electrode and the third electrode are detachably connected to corresponding ends of the two heating members, respectively, the two heating members, the second electrode and the third electrode are collectively enclosed to form the mounting groove.

**14.** The atomizer according to claim 13, wherein the second electrode is provided with at least one second mounting hole, the third electrode is provided with at least one third mounting hole, the at least one second mounting hole and the at least one third mounting hole are detachably coupled to the corresponding ends of the two heating members by fasteners, respectively.

**15.** The atomizer according to claim 13, the base assembly further includes a first electrode, a first insulating member and a second insulating member, a second mounting tube extends downward from the bottom surface of the mounting seat and is in communication with the inner cavity of the mounting seat, the first electrode is mounted in the second mounting tube, and the first electrode is insulated from the mounting seat by the first insulating member, the second electrode is electrically connected with the first electrode, and the second electrode is insulated from the mounting seat by the second insulating member, the third electrode is electrically connected to the mounting seat.

**16.** The atomizer according to claim 15, wherein the second electrode has a "L"-shaped structure, a horizontal edge of the second electrode is detachably sleeved on the upper end of the first electrode and electrically connected to the first electrode, the third electrode is fixedly disposed on the upper surface of the mounting seat with respect to a longitudinal edge of the second electrode.

**17.** The atomizer according to any one of claims 12-16, wherein the side wall of the mounting seat is provided with at least one fourth intake hole in communication with the atomizing chamber, a third intake hole(s) corresponding to the at least one fourth intake hole is defined in the sidewall of the lower portion of the housing, the third intake hole(s) are respectively in communication with the outside and the corresponding fourth intake hole(s).

**18.** The atomizer according to any one of claims 12-16, wherein the atomizer further includes a mouthpiece connector, the mouthpiece connector is detachably mounted at the upper end of the housing, the internal space of the mouthpiece connector forms an air outlet passage, the air outlet passage is in communication with the atomizing chamber.

**19.** The atomizer according to claim 18, wherein the upper end of the housing extends inward in the radial direction of the housing to form an abutting portion, the end of the abutting portion extends downward in the axial direction of the housing to form a second connecting portion, the lower portion of the mouthpiece connector is necked to form a mounting portion, the constricted neck portion of the mouthpiece connector forms a constricted neck end surface, the mounting portion is detachably coupled to the second connecting portion, the abutting portion abuts against the constricted neck end surface.

**20.** An electronic cigarette, comprising an atomizer according to any one of claims 11-19.

**21.** An atomizing assembly for mounting on an atomizer of an electronic cigarette, wherein the atomizing assembly includes a mounting seat, a liquid absorbing member, a heating member and a fixing member, the mounting seat includes a main body portion, and a second electrode and a third electrode spaced apart from each other and protruded on the same surface of the main body portion, both the second electrode and the third electrode are detachably connected to the fixing member after passing through the liquid absorbing member and the heating member in sequence.

**22.** The atomizing assembly according to claim 21, wherein the second electrode and the third electrode are spaced apart from each other and protruded on the top surface of the main body portion, respectively, the liquid absorbing member includes a first lower surface in contact with the top surface of the main body portion, a first upper surface opposite to the first lower surface and two receiving holes extending through the first lower surface and the first upper surface, the heating member includes a second lower surface in contact with the first upper surface, a second upper surface opposite to the second lower surface and two through holes extending through the second lower surface and the second upper surface, both the second electrode and the third electrode are detachably connected to the fixing member after sequentially passing through the corresponding receiving holes and the through holes.

**23.** The atomizing assembly according to claim 22, wherein the mounting seat further includes a second liquid inlet groove, the second liquid inlet groove extends through the top surface and the bottom surface of the main body portion, and the second liquid inlet groove is in communication with the liquid absorbing member.

**24.** The atomizing assembly according to claim 23, wherein the second electrode and the third electrode are disposed along the periphery of the main body portion, and the third electrode and the second electrode are symmetrically disposed along the radial direction of the main body portion, the second liquid inlet groove is provided in the direction perpendicular to the line connecting the second electrode and the third electrode.

**25.** The atomizing assembly according to any one of claims 22-24, wherein the mounting seat further includes a first electrode and a first insulating member, the third electrode is electrically connected to the main body portion, the inner side of one end of the second electrode facing the main body portion is provided with an accommodating cavity, one end of the first electrode is received in the accommodating cavity after passing through the top surface of the main body portion and electrically connected to the second electrode, the opposite end of the first electrode passes through the main body portion and protrudes outside the bottom surface of the main body portion, the first insulating member is disposed between the main body portion and the first electrode.

**26.** The atomizing assembly according to any one of claims 21-24, wherein the heating member is provided with at least one smoke outlet groove for emitting smoke.

**27.** The atomizing assembly according to any one of claims 21-24, wherein the number of the fixing member is two, the second electrode and the third electrode are respectively detachably connected to the corresponding fixing member by threads.

**28.** The atomizing assembly according to any one of claims 21-24, wherein the heating member is a stainless steel heating sheet.

**28.** The atomizing assembly according to any one of claims 21-24, wherein the atomizing assembly further includes a sleeve, the sleeve is provided with a receiving cavity, the mounting seat, the liquid absorbing member and the heating member are all received in the receiving cavity.

**30.** An atomizer, comprising an atomizing assembly according to any one of claims 1-9.

**31.** An electronic cigarette, comprising an atomizer according to claim 10.
